**Europäisches Patentamt**

(19) **European Patent Office**

**Office européen des brevets**

(11) Veröffentlichungsnummer: **0 027 187**
**B1**

(12) **EUROPÄISCHE PATENTSCHRIFT**

(45) Veröffentlichungstag der Patentschrift:
**11.07.84**

(21) Anmeldenummer: **80105680.5**

(22) Anmeldetag: **22.09.80**

(51) Int. Cl.³: **A 61 N 5/06**

(54) **Bräunungsgerät für Direktpigmentierung.**

(30) Priorität: **12.10.79 DE 2941467**
**07.07.80 DE 3025688**

(43) Veröffentlichungstag der Anmeldung:
**22.04.81 Patentblatt 81/16**

(45) Bekanntmachung des Hinweises auf die Patenterteilung:
**11.07.84 Patentblatt 84/28**

(84) Benannte Vertragsstaaten:
**AT BE CH DE FR GB IT LI NL SE**

(56) Entgegenhaltungen:
**DE - A - 1 464 661**
**DE - A - 2 454 169**
**DE - A - 2 537 855**
**DE - A - 2 605 487**
**DE - A - 2 607 249**
**DE - A - 2 609 273**
**DE - A - 2 714 724**
**DE - A - 2 823 615**
**GB - A - 1 468 556**
**GB - A - 2 012 939**

(73) Patentinhaber: **Sellmeier, Hans Robert, Dr.,
Gerhart-Hauptmann-Strasse 48, A-6020 Innsbruck (AT)**

(72) Erfinder: **Sellmeier, Hans Robert, Dr.,
Gerhart-Hauptmann-Strasse 48, A-6020 Innsbruck (AT)**

(74) Vertreter: **Körber, Wolfhart, Dr. et al, Patentanwälte
Dipl.-Ing. H. Mitscherlich Dipl.-Ing. K. Gunschmann
Dr.rer.nat. W. Körber Dipl.Ing. J. Schmidt-Evers Dipl.-Ing.
W. Melzer Steinsdorfstrasse 10, D-8000 München 22 (DE)**

## Beschreibung

Die Erfindung bezieht sich auf ein Bräunungsgerät zur Direktpigmentierung der menschlichen Haut, mit mindestens einem in einem gemeinsamen Gehäuse angeordneten Hg-Hochdruckstrahler, mindestens einem Wärmefilter, mindestens einem UV-Filter, einem Reflektor, sowie mit einem Gebläse zur Zuführung von Kühlluft.

Es ist bekannt, dass nur Licht eines ganz bestimmten Wellenlängenbereiches eine erwünschte Bräunung der menschlichen Haut herbeiführt. Die äussere Wirkung einer UV-Strahlung auf die menschliche Haut zeigt sich in einer Erythembildung (Sonnenbrand), einer nachfolgenden sekundären Pigmentierung, einer sogenannten Lichtschwiele, einer direkten Pigmentierung, sowie in ungünstigen Fällen in weiteren chronischen Lichtschäden bis hin zu solaren Verbrennungen und Hautkrebs. Das UV-Erythem tritt im Gegensatz zum Wärmeerythem nicht unmittelbar nach der Bestrahlung ein, sondern erst nach einer Latenzzeit von einigen Stunden.

Der zeitliche Verlauf der Rötung ist individuell, lokal und von der spektralen Verteilung der UV-Strahlung abhängig. Es kann als wissenschaftlich gesichert gelten, dass die Erythemwirksamkeitsschwelle bei einer Bestrahlung von 200–600 Ws/m² bei einer Wellenlänge von 297 nm erreicht wird, (1) Dissertation Dipl.-Ing. Bernhard Steck, Berlin 1975, Technische Universität.

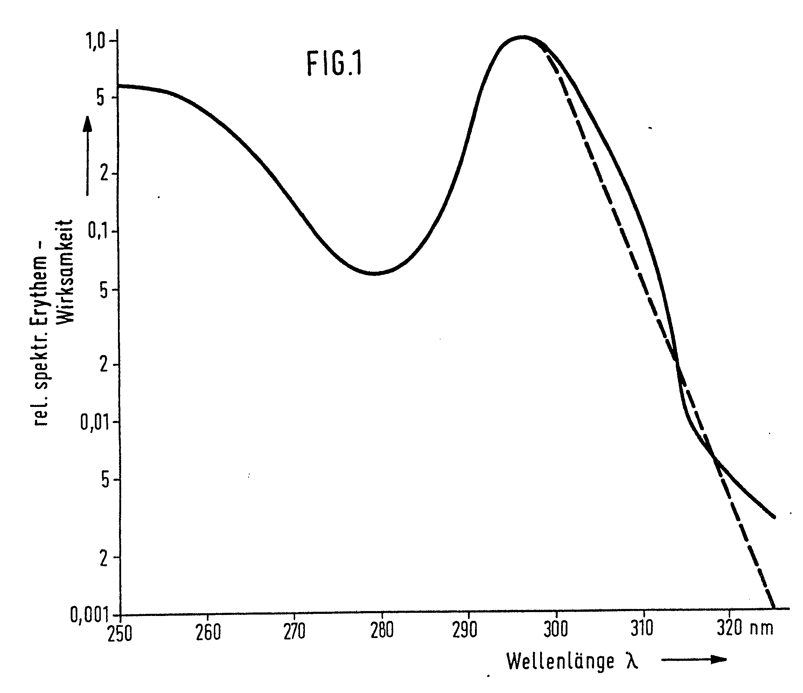

Bei einer Bestrahlung der menschlichen Haut mit UV-Licht tritt je nach Höhe der Bestrahlung eine mehr oder weniger ausgeprägte, auf die bestrahlten Stellen begrenzte sekundäre Pigmentierung, d.h. Hautbräunung ein. Die sekundäre Pigmentierung hat eine sehr lange Latenzzeit. Die spektrale Wirkungsfunktion der Bildung des sekundären Pigments ist derjenigen der Erythembildung grundsätzlich gleich. In Fig. 1 ist die aus (1) als gesichert zu entnehmende spektrale Erythemwirksamkeitskurve dargestellt.

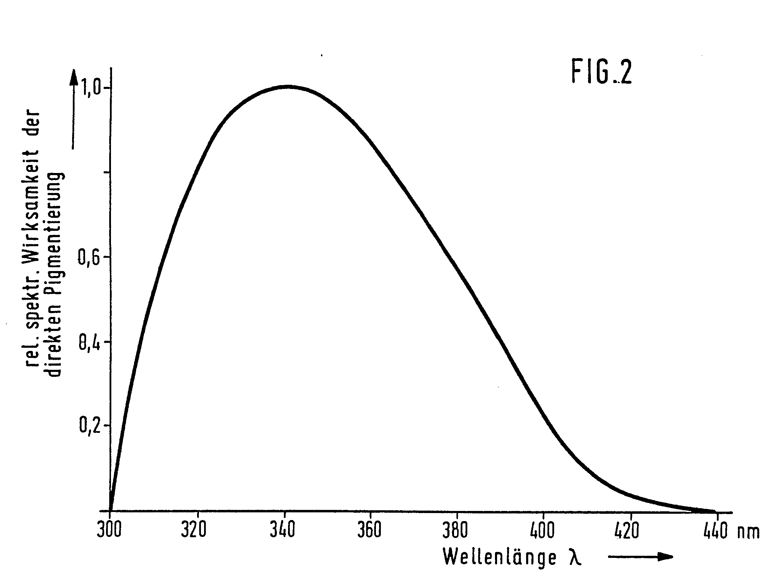

Zu unterscheiden von der sekundären, nicht notwenigerweise einem Erythem folgenden Pigmentierung, die vor allem durch Strahlung unterhalb 310 nm hervorgerufen wird, ist die direkte Pigmentierung (Hautbräunung), die ohne ein vorausgehendes Erythem innerhalb weniger Minuten entsteht und im wesentlichen von UV-A-Strahlung erzeugt wird. Gemäss Fig. 2 hat die spektrale Wirkungskurve der direkten Pigmentierung bei 340 nm ein breites Maximum und verläuft bis in den sichtbaren Wellenlängenbereich. Nach (1) ist im Gebiet maximaler Empfindlichkeit bei 340 nm zum Erreichen der Direktpigmentierungsschwelle im Mittel eine Bestrahlung von etwa 100–200 kWs/m² notwendig. Dies ist ersichtlich eine beträchtlich höhere Dosis als die vorstehend für die Erythemschwelle angegebene Dosis von 0,2 bis 0,6 kWs/m² bei 297 nm.

Für die Konstruktion eines Bräunungsgerätes zur Direktpigmentierung kommt es somit darauf an, die Direktpigmentierungsschwelle durch Bestrahlung im entsprechenden Wellenlängenbereich zu erreichen und zu überschreiten, ohne dabei die Erythemschwelle im dafür angegebenen Wellenlängenbereich zu überschreiten. Damit werden erhebliche Forderungen an die Filterauswahl und deren Kühlung sowie den geeigneten Betrieb und die Auswahl des entsprechend dotierten Hg-Hochdruckstrahlers gestellt.

Erwähnt sei noch, dass die durch die angestrebte direkte Pigmentierung hervorgerufene Bräunung der Haut eine rötlich-braune Färbung ist, welche beständiger ist, als die vorerwähnte sekundäre Pigmentierung, die einem Erythem folgt.

Der Erfindung liegt die Aufgabe zugrunde, ein Bräunungsgerät der eingangs genannten Art zu schaffen, bei dem die Filter so ausgebildet und ausgewählt werden können und mit dem Strahler so angeordnet sind, dass eine gezielte, gegebenenfalls unterschiedliche Kühlung der verschiedenen Geräteteile bei gewünschter Leistung erreicht wird.

Zur Lösung dieser Aufgabe wird erfindungsgemäss vorgeschlagen, dass das Gehäuse durch eine Zwischenwand in zwei Gehäusebereiche unterteilt ist, einem ersten Gehäusebereich, der mit der Luftansaugseite des Gebläses verbunden ist und einem zweiten Gehäusebereich, der mit der Druckseite des Gebläses verbunden ist, und dass der/die Hg-Hochdruckstrahler in einem der Gehäusebereiche angeordnet sind, wobei das Wärmefilter und das UV-Filter im unmittelbaren Luftstrom der angesaugten und/oder ausgeblasenen Kühlluft liegen und zumindest der Entladungsteil des Hg-Hochdruckstrahlers vom angesaugten und/oder ausgeblasenen Kühlluftstrom abgeschirmt ist.

Eine erste Ausgestaltung der Erfindung ist dadurch gekennzeichnet, dass bei nur einem in dem Gehäuse 1 angeordneten Hg-Hochdruckstrahler 2 dieser im zweiten, mit der Druckseite des Gebläses 12 verbundenen Gehäusebereich 8 angeordnet ist, dass das Wärmefilter 5 in einer Aussenwand des Gehäuses 1 die Abstrahlöffnung des Gerätes überdeckend angeordnet ist und diese Aussenwand mit Wärmefilter den ersten Gehäusebereich 7 auf der einen Seite abgrenzt, das UV-Filter 9 in der Zwischenwand 6 angeordnet ist, und dass der Reflektor 18 unterteilt ist und sich sowohl im ersten 7 als auch im zweiten Gehäusebereich 8 erstreckt und in seinem in dem zweiten Gehäusebereich 8 liegenden Teil 20 eine Eintrittsöffnung 21 und eine Austrittsöffnung 22 aufweist, die ein Vorbeiströmen des ausgeblasenen Kühlluftstromes an dem UV-Filter 9 zulassen.

Ein mit nur einem Hg-Hochdruckstrahler ausgerüstetes Bräunungsgerät ist in seiner Leistung, insbesondere aber in der Grösse der zu bestrahlenden Fläche begrenzt. Eine weitere Aufgabe der Erfindung ist somit darin zu sehen, ein grosses Bräunungsgerät mit mehreren in einem Gehäuse angeordneten Hg-Hochdruckstrahlern zu schaffen, welche zur Bestrahlung eines ganzen, beispielsweise auf einer Liege ausgestreckten menschlichen Körpers von Kopf bis Fuss geeignet ist.

Zwar könnten bei der vorstehend beschriebenen ersten Ausgestaltung der Erfindung mehrere Hg-Hochdruckstrahler in einem Gehäuse nebeneinander angeordnet und entsprechend der dort beschriebenen Kühlluftführung gekühlt werden. Eine genügend gleichmässige Kühlung für mehrere beispielsweise nebeneinander angeordnete Hg-Hochdruckstrahler stösst jedoch auf gewisse Schwierigkeiten, da das Hindurchführen des druckseitigen, von dem Gebläse ausgeblasenen Kühlluftstromes durch die Reflektoren mehrerer Strahler hintereinander nicht möglich ist. Eine gleichzeitige unmittelbare Versorung mehrerer Refklektoren setzt eine sehr grosse druckseitige Austrittsöffnung des Gebläses voraus.

Als zweckmässige zweite Ausgestaltung der Erfindung wird daher vorgeschlagen, dass bei mehreren in dem Gehäuse angeordneten Hg-Hochdruckstrahlern diese in einer Reihe nebeneinander im ersten, mit der Luftansaugseite des Gebläses verbundenen Gehäusebereich angeordnet sind, dass das Wärmefilter in einer Aussenwand des Gehäuses, die Abstrahlöffnung des Gerätes überdeckend angeordnet ist und diese Aussenwand mit Wärmefilter den zweiten, mit der Druckseite des Gebläses verbundenen Gehäusebereich abgrenzt, je ein UV-Filter für jeden Strahler in der Zwischenwand angeordnet ist, dass jeder Hg-Strahler von einem ersten, zumindest teilweise parabolförmigen an der Zwischenwand befestigten Reflektor umgeben ist, der den Entladungsteil des Hg-Hochdruckstrahlers gegen den angesaugten Kühlluftstrom abschirmt, und dass sich im zweiten Gehäusebereich ein zweiter, rinnenförmiger und allen Strahlern gemeinsamer Reflektor zwischen den UV-Filtern und dem Wärmefilter erstreckt und in seiner Längsrichtung vom ausgeblasenen Kühlluftstrom durchströmt wird.

Gegenüber den mit nur einem Hg-Strahler ausgerüsteten Bräunungsgerät nach der ersten Ausführungsform bestehen die wesentlichen Unterschiede darin, dass die mehreren Hg-Hochdruckstrahler im ersten, mit der Luftansaugseite des Gebläses verbundenen Gehäusebereich angeordnet sind und das gemeinsame Wärmefilter und die in der Zwischenwand angeordneten UV-Filter durch den druckseitigen, d.h. ausgeblasenen Kühlluftstrom des Gebläses im zweiten Gehäusebereich gekühlt werden. Jeder Strahler ist von einem ersten, an der Zwischenwand befestigten Reflektor umgeben und durch diesen zumindest in seinem Entladungsbereich gegen den unmittelbaren Kühlluftstrom abgeschirmt. Trotzdem erfolgt eine gute intensive Kühlung der ersten Reflektoren von deren Aussenseite in dem den Strahler unmittelbar umgebenden Bereich. Der gemeinsame rinnenförmige Reflektor im zweiten Gehäusebereich dient gleichzeitig als Luftführung.

In zweckmässiger Weiterbildung der zweiten Ausgestaltung der Erfindung ragen die die elektrischen Anschlüsse jedes Hg-Hochdruckstrahlers tragenden Quetschfüsse seitlich über jeden zugehörigen ersten Reflektor nach aussen und werden von dem angesaugten, aussen am ersten Reflektor vorbeistreichenden angesaugten Kühlluftstrom intensiv gekühlt.

Eine besonders intensive Kühlung der die Hg-Hochdruckstrahler jeweils unmittelbar umgebenden Bereiche der ersten Reflektoren ist in weiterer Ausbildung der zweiten Ausgestaltung der Erfindung dadurch gewährleistet, dass im ersten Gehäusebereich ein Luftansaugkanal abgeteilt ist, der Luftansaugöffnungen aufweist und der mit der Saugseite des Gebläses verbunden ist in welchen die den Hg-Hochdruckstrahler jeweils unmittelbar umgebenden Bereiche der ersten Reflektoren ragen. Dabei kann zweckmässig jedem Strahler ein oder mehrere getrennte Luftansaugöffnungen zugeordnet sein. Gleichzeitig kann in dem Luftansaugkanal das zu jedem Strahler gehörende Zündgerät angeordnet sein, so dass auch dieses in gewünschter Weise vom Strom der angesaugten Kühlluft gekühlt wird.

Die Ausbildung des UV-Filters aus sich schuppenartig überlappenden Streifen aus Blauviolett-Filterglas, wobei sich die Streifen des Filterglases in Richtung des vorbeistreichenden Kühlluftstromes erstrecken, nach den Ansprüchen 14 und 15, hat den besonderen Vorteil, dass die für diesen Wellenlängenbereich mit einer Absorptionskante bei 320–310 nm besonders empfindlichen Filtergläser ungenügend gekühlt werden können, ohne dass ein Verziehen oder Platzen oder anderweitige Änderungen des Transmissionsgrades zu befürchten sind.

Beispielsweise Ausführungsformen der Erfindung werden nachstehend anhand der Fig. 3 bis 7 beschrieben. Es zeigen

Fig. 1 die Erythemwirksamkeitskurve;

Fig. 2 die spektrale Wirkungskurve der direkten Pigmentierung;

Fig. 3 schematisch in einer vertikalen Schnittansicht ein Bräunungsgerät nach erster Ausgestaltung der Erfindung;

Fig. 4 eine Schnittansicht entlang der Linie IV–IV in Fig. 3;

Fig. 5 eine schematische Ansicht des Bräunungsgerätes im Längsschnitt nach der zweiten Ausgestaltung der Erfindung;

Fig. 6 eine Schnittansicht entlang der Linie II–II in Fig. 1 und

Fig. 7 eine Schnittansicht entlang der Linie III–III in Fig. 5.

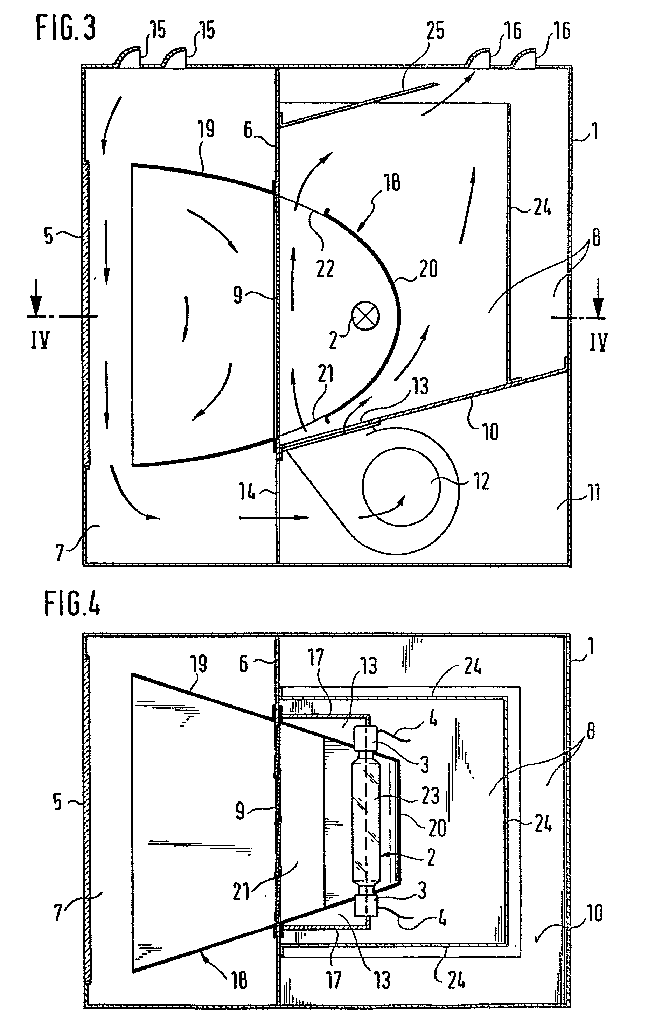

Das Bräunungsgerät nach der ersten Ausgestaltung der Erfindung (Fig. 3 und 4) umfasst ein Gehäuse 1 mit einem darin angeordneten Hg-Hochdruckstrahler 2, wobei dessen elektrische Anschlüsse an den in Fig. 4 ersichtlichen Quetschfüssen 3 nur schematisch bei 4 angedeutet sind. Das Gehäuse 1 weist an seiner Vorderseite in einer Strahlungsaustrittsöffnung ein Wärmefilter 5 auf und ist durch eine Zwischenwand 6 in einen ersten Gehäusebereich 7 und einen zweiten Gehäusebereich 8 unterteilt. In der Zwischenwand 6 ist ein UV-Filter 9 angeordnet, welches aus Streifen von geeignetem Blauviolett-Filterglas zusammengesetzt ist. Durch eine Trennwand 10 ist vom zweiten Gehäusebereich 8 eine Kammer 11 abgetrennt, in welcher sich ein Lüftergebläse 12 befindet. In der Trennwand 10 ist eine druckseitige Gebläseöffnung 13 vorgesehen, während in der Zwischenwand 6 eine Ansaugöffnung 14 für das Gebläse 12 vorgesehen ist. Auf der der Ansaugöffnung 14 gegenüberliegenden Seite des ersten Gehäusebereiches sind Luftansaugschlitze 15 angeordnet; entsprechend sind im zweiten Gehäusebereich 8 auf der der Gebläseöffnung 13 gegenüberliegenden Seite Auslassschlitze 16 vorgesehen.

Neben dem Filter 9 ist an der Zwischenwand 6 über Arme 17 der Hochdruckstrahler 2 befestigt. Weiter ist an der Zwischenwand 6 ein Reflektor 18 aufgehängt, welcher aus zwei Teilen besteht, einem sich im ersten Gehäusebereich 7 erstreckenden Teil 19, welcher in genügendem Abstand vor der den Wärmefilter 5 tragenden Aussenwand des Gehäuses 1 endet, um ein Durchtreten des durch die Ansaugschlitze 15 und die Ansaugöffnung 14 vom Gebläse 12 angesaugten Kühlluftstromes zu ermöglichen. Der andere, im zweiten Gehäusebereich 8 liegende Teil 20 des Reflektors weist eine Eintrittsöffnung 21 und eine Austrittsöffnung 22 für den durch die druckseitige Gebläseöffnung 13 ausgeblasenen Kühlluftstromes auf, so dass dieser Kühlluftstrom unmittelbar an dem UV-Filter 9 vorbeistreichen kann. Gleichzeitig liegt der Entladungsteil 23 des Hochdruckstrahlers 2 innerhalb des Strömungsschattens des parabolisch ausgebildeten Reflektorteiles 20, während, wie aus Fig. 4 ersichtlich, die Quetschfüsse 3 des Strahlers 2 über den Reflektorteil 20 hinausragen und im unmittelbaren Kühlluftstrom liegen. Im zweiten Gehäuseteil 8 sind an der Trennwand 10 ein Leitblech 24 und an der Zwischenwand 6 ein Leitblech 25 verstellbar befestigt, um den ausgeblasenen Kühlluftstrom hinsichtlich der durch und hinter dem Reflektor vorbeiströmenden Luftmenge einstellen zu können und den Kühlluftstrom zu den Auslassschlitzen 16 zu leiten.

Während des Betriebes des Bräunungsgerätes wird Raumluft durch das Gebläse 12 angesaugt. Sie tritt durch die Ansaugschlitze 15 in das Gerät ein und streicht unmittelbar an dem Wärmefilter 5 vorbei, wobei ein geringer Anteil dieser Luft auch innerhalb des Reflektorteiles 19 den UV-Filter 9 erreicht und kühlt. Der aus der druckseitigen Gebläseöffnung 13 austretende Luftstrom ist so gerichtet, dass das UV-Filter 9 optimal durch die durch die Eintrittsöffnung 21 und die Austrittsöffnung 22 des Reflektorteiles 20 hindurchtretende Luft gekühlt wird. Der Entladungsteil 23 des Strahlers 2 liegt dabei im Strömungsschatten des parabolischen Reflektorteiles 20 und wird vom Kühlluftstrom wenig getroffen, so dass seine optimale Betriebstemperatur, die zwischen 700–900 °C liegt, nicht gemindert wird. Die Gebläseöffnung 13 ist so gelegt und dimensioniert, dass der Kühlluftstrom auch seitwärts aussen am Reflektorteil 20 vorbeistreicht und damit die Quetschfüsse 3 des Strahlers 2 wirksam gekühlt werden, so dass erreicht wird, dass deren Temperatur nicht über 350 °C steigt. Die Wirkung dieses Kühlluftstromes wird durch entsprechende Einstellung der Leitbleche 24 erhöht.

Das UV-Filter 9 ist aus sich überlappenden Blauviolettglas-Streifen zu dem Filter 9 in jeder beliebigen Filtergrösse zusammengesetzt. Dieses Filter 9 ist soweit von dem Strahler 2 entfernt und wird so wirksam gekühlt, dass die Temperatur des Glases 200 °C nicht überschreitet. Andernfalls würde sich der Transmissionsgrad dieses Glases verändern. Das Wärmefilter 5 ist ein handelsübliches Wärmeabsorptionsglas. Durch Variation der Dicke der Filtergläser wird der gewünschte Transmissionsgrad eingestellt, wobei die erfindungsgemässe Konstruktion des Bräunungsgerätes insbesondere wegen seiner optimalen Kühlung dem Anwender einen weiten Variationsbereich für die Filterwahl lässt. Die Filterkombination soll so ausgelegt sein, dass nur UV-A-Strahlen (315–400 nm) und sichtbares Licht bis etwa 450 nm hindurchgelassen werden, da in diesem Bereich die Spektralwirkungskurve der direkten Pigmentierung liegt (Fig. 2). Die kurzwellige Grenze der Strahlung wird durch die Erythemwirksamkeitskurve (Fig. 1) vorgegeben. Die Filter des beschriebenen Bräunungsgerätes sollen so ausgelegt sein, dass die Erythemschwellenzeit oberhalb von 100 Std. liegt, wobei die ungünstigere der Fig. 1 angegebenen Kurven angenommen werden soll. Das heisst bei einem ausgewählten Hochdruckstrahler mit geringerer Bestrahlungsstärke im erythemwirksamen Bereich kann eine Filterkombination verwendet werden, die schon Strahlen zwischen 315 und 320 nm durchlässt. Hat dagegen der verwendete Strahler eine grössere Bestrahlungsstärke im erythemwirksamen Bereich, so darf die Filterkombination erst langwelligere Strahlen (etwa im Bereich von 320–325 nm) hindurchlassen, damit die Forderung, dass die Erythemschwellendosis frühestens nach 100 Std. erreicht wird, sichergestellt ist.

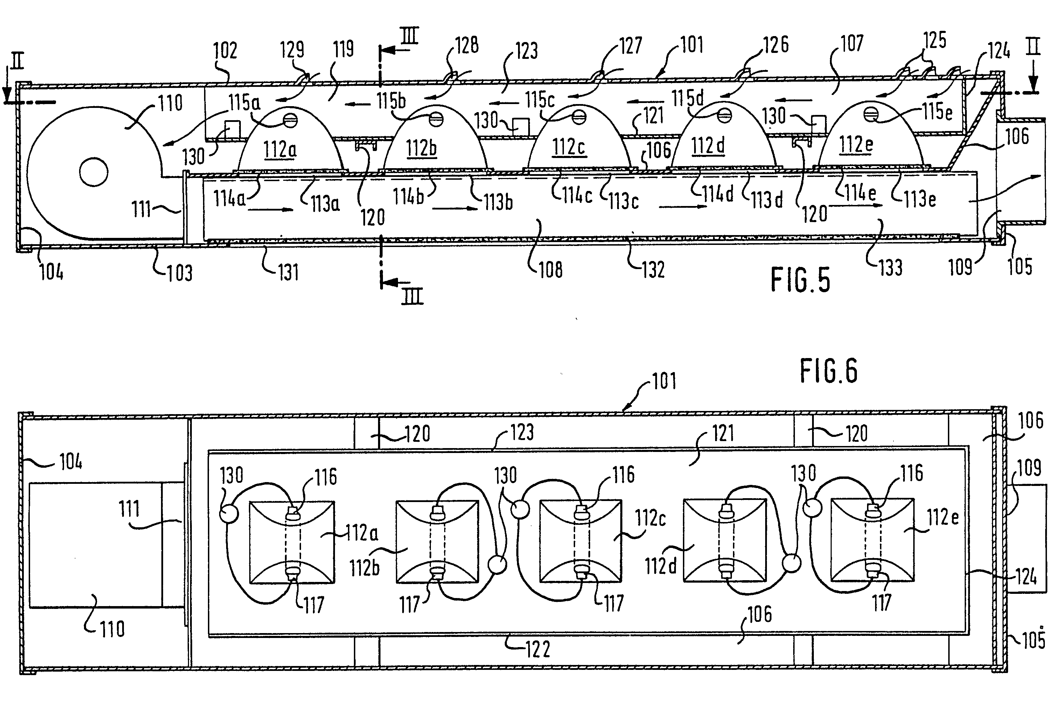

Das in Fig. 5–7 gezeigte Bräunungsgerät nach zweiter Ausgestaltung der Erfindung mit mehreren Hg-Hochdruckstrahlern besteht aus einem insgesamt mit 101 bezeichneten Gehäuse, welches durch eine obere Längswand 102 und eine untere Längswand 103 begrenzt ist, sowie durch Stirnwände 104 und 105. Das Gehäuse 101 ist durch eine Zwischenwand 106 in einen ersten oberen Gehäusebereich 107 und einen zweiten unteren Gehäusebereich 108 unterteilt. Die Zwischenwand 106 ist in Fig. 1 rechts zur Stirnwand 105 hin nach oben gezogen, so dass der zweite, untere Gehäusebereich 108 mit einer in der Stirnwand 105 vorgesehenen Luftaustrittsöffnung 109 in Verbindung steht. An dem in Fig. 5 linken Ende des Gehäuses ist ein Gebläse 110 angeordnet, dessen im einzelnen nicht dargestellte Ansaugöffnungen mit dem ersten Gehäusebereich 107 in Verbindung stehen und dessen druckseitige Austrittsöffnung 111 mit dem zweiten, unteren Gehäusebereich 108 verbunden ist.

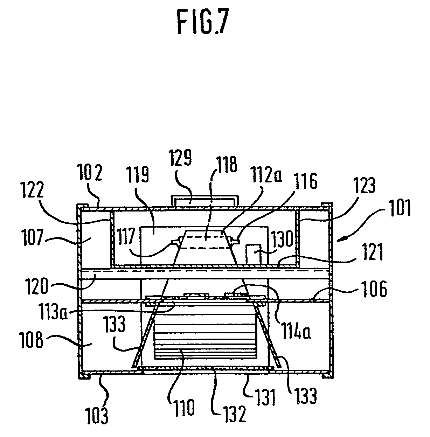

An der Zwischenwand 106 sind in Reihe nebeneinander eine Anzahl, bei der dargestellten Ausführungsform 5 erste Reflektoren 112a bis 112e angeordnet, deren Austrittsöffnungen über Lichtdurchtrittsöffnungen 113a bis 113e liegen, in denen jeweils ein UV-Filter 114a bis 114e angeordnet ist. Innerhalb jedes der ersten Reflektoren 112a bis 112e ist ein Hg-Hochdruckstrahler 115a bis 115e angeordnet, und zwar derart, dass, wie insbesondere aus Fig. 6 und 7 ersichtlich, seine jeweiligen Quetschfüsse mit den elektrischen Anschlüssen 116 und 117 über den Reflektor hinausragen und dem Kühlluftstrom zugänglich sind, während die Entladungsbereiche 118 (Fig. 7) innerhalb der Reflektoren, gegen den Kühlluftstrom abgeschirmt liegen.

Zur konzentrierten Kühlluftführung und intensiven Kühlung nur der die Hg-Hochdruckstrahler 115a bis 115e unmittelbar umgebenden Bereiche der ersten Reflektoren 112a bis 112e ist im ersten Gehäusebereich 107 ein Luftansaugkanal 119 abgeteilt, bestehend aus der an Traversen 120 befestigten Bodenwand 121 mit jeweils Öffnungen für den Durchtritt der ersten Reflektoren, den Seitenwänden 122, 123 (Fig. 6 und 7) und der Stirnwand 124. In der oberen Gehäusewand 102 sind Luftansaugöffnungen 125, 126, 127, 128, 129 vorgesehen, die in den Luftansaugkanal 119 münden. Ersichtlich sind jedem Strahler Luftansaugöffnungen zugeordnet, so dass die von dem Gebläse 110 durch den Kanal 119 angesaugte Kühlluft von den Luftansaugöffnungen 125 bis 129 unmittelbar an den Rückseiten der ersten Reflektoren 112a bis 112e und den intensiv zu kühlenden Quetschfüssen und elektrischen Anschlüssen 116, 117 der Strahler vorbeistreicht und diese intensiv kühlt. Zweckmässig sind innerhalb des Luftansaugkanals 119 auf dessen Bodenwand 121 die Zündgeräte 130 für jeden Hg-Hochdruckstrahler angeordnet, um diese ebenfalls wunschgemäss zu kühlen.

Der zweite untere Gehäusebereich 108 ist durch die Gehäusewand 103 begrenzt, welche die Austrittsöffnung 131 für das Bestrahlungslicht aufweist. Vor dieser Austrittsöffnung befindet sich das Wärmefilter 132, welches sich einstückig über die gesamte Öffnung 131 erstrecken kann oder auch aus mehreren Teilen zusammengesetzt sein kann. Innerhalb des zweiten Gehäusebereiches 108 ist zwischen den UV-Filtern 114a bis 114e und dem Wärmefilter 132 ein zweiter, sich nach unten rinnenförmig öffnender Reflektor 133 angeordnet, der für alle Strahler gemeinsam vorgesehen ist und gleichzeitig als Führungskanal für die vom Gebläse 110 durckseitig ausgeblasene Kühlluft dient, um diese an den UV-Filtern und dem Wärmefilter unter deren intensiver Kühlung entlang zur Austrittsöffnung 109 zu leiten.

Insgesamt ist der Verlauf des Kühlluftstromes durch das Bräunungsgerät durch Pfeile angedeutet. Ersichtlich werden durch die beschriebene konstruktive Ausführung des Gerätes und die Führung des Kühlluftstromes die intensiv zu kühlenden Teile wie Wärmefilter, UV-Filter und die die Hg-Hochdruckstrahler unmittelbar umgebenden Bereiche der ersten Reflektoren sowie die Quetschfüsse und elektrischen Anschlüsse der Hg-Hochdruckstrahler intensiv unmittelbar gekühlt, während die Entladungsbereiche 118 der Hg-Hochdruckstrahler vom Kühlluftstrom durch die Reflektoren abgeschirmt sind. Die elektrischen Versorgungsteile und Versorgungsleitungen zu den Hg-Hochdruckstrahlern bzw. den Zündgeräten 130 sind nicht näher dargestellt. Sie brauchen nicht innerhalb des schematisch dargestellten Bräunungsgerätes angeordnet zu sein. Vielmehr können die elektrischen Versorgungsteile beispielsweise in dem nicht dargestellten Stativ des Bräunungsgerätes oder sogar unterhalb des Liegebettes angeordnet sein, welches unter der Austrittsöffnung 131 für das Bestrahlungslicht steht.

**Patentansprüche**

1. Bräunungsgerät zur Direktpigmentierung der menschlichen Haut mit mindestens einem in einem gemeinsamen Gehäuse angeordneten Hg-Hochdruckstrahler, mindestens einem Wärmefilter, mindestens einem UV-Filter, einem Reflektor, sowie mit einem Gebläse zur Zuführung von Kühlluft, dadurch gekennzeichnet, dass das Gehäuse (1, 101) durch eine Zwischenwand (6, 106) in zwei Gehäusebereiche unterteilt ist, einen ersten Gehäusebereich (7, 107), der mit der Luftansaugseite des Gebläses (12) verbunden ist, und einen zweiten Gehäusebereich (8, 108), der mit der Druckseite des Gebläses (12, 110) verbunden ist, und dass der/die Hg-Hochdruckstrahler (2, 115) in einem der Gehäusebereiche angeordnet sind, wobei das Wärmefilter (5, 132) und das UV-Filter (9, 114) im unmittelbaren Luftstrom der angesaugten und/oder ausgeblasenen Kühlluft liegen und zumindest der Entladungsteil (23, 118) des Hg-Hochdruckstrahlers (2, 115) vom angesaugten und/oder ausgeblasenen Kühlluftstrom abgeschirmt ist.

2. Bräunungsgerät nach Anspruch 1, dadurch gekennzeichnet, dass bei nur einem in dem Gehäuse (1) angeordneten Hg-Hochdruckstrahlers (2) dieser im zweiten, mit der Druckseite des Gebläses (12) verbundenen Gehäusebereich (8) angeordnet, dass das Wärmefilter (5) in einer Aussenwand des Gehäuses (1) die Abstrahlöffnung des Gerätes überdeckend angeordnet ist und diese Aussenwand mit Wärmefilter den ersten Gehäusebereich (7) auf der einen Seite abgrenzt, das UV-Filter (9) in der Zwischenwand (6) angeordnet ist, und dass der Reflektor (18) unterteilt ist und sich sowohl im ersten (7) als auch im zweiten Gehäusebereich (8) erstreckt und in seinem in dem zweiten Gehäusebereich (8) liegenden Teil (20) eine Eintrittsöffnung (21) und eine Austrittsöffnung (22) aufweist, die ein Vorbeiströmen des ausgeblasenen Kühlluftstromes an dem UV-Filter (9) zulassen.

3. Bräunungsgerät nach Anspruch 2, dadurch gekennzeichnet, dass der Reflektor (18) zumindest in einer Schnittebene parabolisch ausgebildet ist und seine Eintrittsöffnung (21) derart vor der druckseitigen Gebläseöffnung (13) liegt, dass ein Anteil des ausgeblasenen Kühlluftstromes hinter dem Reflektor vorbeistreicht und der Entladungsteil (23) des Hg-Hochdruckstrahlers (2) im Strömungsschatten des durch die Eintrittsöffnung (21) einströmenden Anteiles des Kühlluftstromes liegt.

4. Bräunungsgerät nach Anspruch 3, dadurch gekennzeichnet, dass die die elektrischen Anschlüsse (4) des Hg-Hochdruckstrahlers (2) tragenden Quetschfüsse (3) seitlich über den Reflektor (18) nach aussen ragen und von dem hinter dem Reflektor vorbeistreichenden Anteil des Kühlluftstromes gekühlt werden.

5. Bräunungsgerät nach Anspruch 2 oder 3, dadurch gekennzeichnet, dass der im ersten Gehäusebereich (7) liegende Teil (19) des Reflektors (18) an der Zwischenwand (6) angeordnet ist und in einem Abstand von der das Wärmefilter (5) tragenden Aussenwand endet und einen Durchlass für den angesaugten Kühlluftstrom freilässt.

6. Bräunungsgerät nach einem der vorhergehenden Ansprüche, dadurch gekennzeichnet, dass das Gebläse (12) innerhalb des Gehäuses (1) in einer von dem zweiten Gehäusebereich (8) durch eine Trennwand (10) abgeteilten Kammer (11) angeordnet ist, wobei die druckseitige Gebläseöffnung (13) in der Trennwand (10) liegt und in der Zwischenwand (6) zwischen dem ersten Gehäusebereich (7) und der Kammer (11) eine Ansaugöffnung (14) vorgesehen ist.

7. Bräunungsgerät nach Anspruch 6, dadurch gekennzeichnet, dass im zweiten Gehäusebereich (8) Leitbleche (24, 25) zur gesteuerten Ableitung des ausgeblasenen Kühlluftstromes zu Auslassschlitzen (16) im Gehäuse (1) hin angeordnet sind.

8. Bräunungsgerät nach Anspruch 7, dadurch gekennzeichnet, dass je ein Leitblech (25) an der Zwischenwand gegenüber der Austrittsöffnung (22) des Reflektors (18) und mindestens ein Leitblech (24) an der Trennwand (10) gegenüber der Rückseite des Reflektors (18) verstellbar befestigt ist.

9. Bräunungsgerät nach Anspruch 1, dadurch gekennzeichnet, dass bei mehreren in dem Gehäuse (101) angeordneten Hg-Hochdruckstrahlern (115a–e) diese in einer Reihe nebeneinander im ersten, mit der Luftansaugseite des Gebläses (110) verbundenen Gehäusebereich (107) angeordnet sind, dass das Wärmefilter (132) in einer Aussenwand (103) des Gehäuses, die Abstrahlöffnung (131) des Gerätes überdeckend angeordnet ist und diese Aussenwand mit Wärmefilter den zweiten, mit der Druckseite des Gebläses (110) verbundenen Gehäusebereich (108) abgrenzt, je ein UV-Filter (114a–e) für jeden Strahler (115a–e) in der Zwischenwand (106) angeordnet ist, dass jeder Hg-Strahler von einem ersten, zumindest teilweise parabolförmigen an der Zwischenwand (106) befestigten Reflektor (112a–e) umgeben ist, der den Entladungsteil (118) des Hg-Hochdruckstrahlers gegen den angesaugten Kühlluftstrom abschirmt, und dass sich im zweiten Gehäusebereich (108) ein zweiter, rinnenförmiger und allen Strahlern gemeinsamer Reflektor (133) zwischen den UV-Filtern (114a–e) und dem Wärmefilter (132) erstreckt und in seiner Längsrichtung vom ausgeblasenen Kühlluftstrom durchströmt wird.

10. Bräunungsgerät nach Anspruch 9, dadurch gekennzeichnet, dass die die elektrischen Anschlüsse jedes Hg-Hochdruckstrahlers (115a–e) tragenden Quetschfüsse (116, 117) seitlich über jeden zugehörigen ersten Reflektor (112a–e) nach aussen ragen und von dem angesaugten, aussen am ersten Reflektor vorbeistreichenden angesaugten Kühlluftstrom gekühlt werden.

11. Bräunungsgerät nach Anspruch 9 und 10, dadurch gekennzeichnet, dass im ersten Gehäusebereich (107) ein Luftansaugkanal (119) abgeteilt ist, der Luftansaugöffnungen (125–129) aufweist und der mit der Saugseite des Gebläses (110) verbunden ist und in welchem die dem Hg-Hochdruckstrahler (115) jeweils unmittelbar umgebenden Bereiche der ersten Reflektoren (112) ragen.

12. Bräunungsgerät nach Anspruch 11, dadurch gekennzeichnet, dass jedem Strahler (115) ein oder mehrere getrennte Luftansaugöffnungen (125–129) zugeordnet sind.

13. Bräunungsgerät nach Anspruch 11 oder 12, dadurch gekennzeichnet, dass das zu jedem Strahler (115) gehörende Zündgerät (130) innerhalb des Luftansaugkanals (119) im Strom der angesaugten Kühlluft angeordnet ist.

14. Bräunungsgerät nach einem der vorhergehenden Ansprüche, dadurch gekennzeichnet, dass das UV-Filter (9) aus sich schuppenartig überlappenden Streifen aus Blauviolett-Filterglas zusammengesetzt ist.

15. Bräunungsgerät nach Anspruch 14, dadurch gekennzeichnet, dass sich die Streifen des Filterglases in Richtung des vorbeistreichenden Kühlluftstromes erstrecken.

## Claims

1. In a suntan apparatus for the direct pigmentation of the human skin including, within a common housing, at least one high-pressure Hg radiator, at least one heat filter, at least one UV-filter, a reflector and a blower for the delivery of cooling air characterised by a partition wall (6, 106) dividing the housing (1, 101) into two housing areas, a first area (7, 107) being connected with the air suction side of the blower (12), and a second area (8, 108) being connected with the pressure side of the blower (12, 110) the Hg-radiator (2, 115) being arranged in one of the housing areas, wherby the heat filter (5, 132) and the UV-filter (9, 114) are located in the direct air flow of the aspirated or expelled cooling air and at least the discharge portion (23, 118) of the Hg radiator (2, 115) is screened off from the aspirated or expelled cooling air flow.

2. Suntan apparatus as claimed in claim 1, characterised by only one of the Hg radiators (2) being arranged in the housing (1) area, which is arranged in the second area (8, 108), being connected with the pressure side of the blower (12, 110), the heat filter (5) being arranged in an outer wall of the housing (1) so as to cover the radiation aperture of the apparatus, the outer wall and heat filter (5) bordering the first area (7) on one side thereof, the UV-filter (9) being arranged in the partition wall (6), the reflector (18) being divided and extending into the first (7) and second area (8) and having an inlet opening (21) and outlet opening (22) in the part located in the second area (8) which facilitate the flow of the expelled cooling air stream past the UV-filter (9).

3. Suntan apparatus as claimed in claim 2, characterised by the reflector (18) being parabolically-shaped in at least one plane, the inlet opening (21) of the reflector (18) being located in front of the pressure-sided blower opening (13) whereby a portion of the expelled cooling air stream passes behind the reflector (18), and the discharge portion (23) of the Hg radiator (2) is located in the quiescent flow portion of the cooling air stream entering through the inlet opening (21).

4. Suntan apparatus as claimed in claim 3, characterised by pinched bases (3) supporting the electrical connections (4) for the Hg radiator (2) projecting sideways outwardly of the reflector (18) so as to be cooled by the portion of the cooling air stream flowing past the reflector (18).

5. Suntan apparatus as claimed in claim 2 or 3 characterised by the part (19) of the reflector (18) located in the first area (7) being arranged on the partition (6) and terminates ata distance from the outer wall supporting the heat filter (5) so as to facilitate a passage for the aspirated cooling air stream.

6. Suntan apparatus as claimed in one of the preceding claims, characterised by a seperating wall (10) dividing a chamber (11) from the second housing area (8), the blower (12) being arranged in the chamber (11), the pressuresided blower opening (13) being in the separating wall (10), and suction opening (14) being provided in the partitionwall (6) intermediate the first housing area (7) and the chamber (11).

7. Suntan apparatus as claimed in claim 6, characterised by guide plates (24, 25) being arranged in the second housing area (8) for the controlled egress of the expelled cooling air stream towards outlet slits (16) formed in the housing (1).

8. Suntan apparatus as claimed in claim 7 characterised by a guide plate (25) fastened to the partition opposite the outlet opening (22) of the reflector (18) and at least one guide plate (20) being adjustably fastened to the separating wall (10) opposite the rear side of the reflector (18).

9. Suntan apparatus as claimed in claim 1, characterised by a plurality of Hg radiators (115a–e) arranged side by side in a row in the first area (107) being connected to the suction side of the blower (110), the heat filter (132) being located in the outer wall (103) of the housing so as to cover the irradiation aperture (131) of the apparatus, the outer wall with the heat filter (32) bordering the second housing area (108), which is connected to the pressure side of the blower (110), one of the UV-filters (114a–e) being provided for each radiator (115a–e) in the partition wall (106), each Hg radiator (115a–e) being encompassed by an at least partially parabole-shaped reflector (112a–e) fastened to the partition (106), the reflectors (112a–e) screening off the discharge portion (118) of the radiator from the aspirated cooling air stream; and a second, through-shaped reflector (133) common to all radiators extending between the UV-filters (114a–e) and the heat filter (132) and being traversed in the longitudinal direction thereof by the expelled cooling air stream.

10. Suntan apparatus as claimed in claim 9, characterised by pinched bases (116, 117) supporting the electrical connections for each Hg radiator (115a–e) projecting sideways outwardly beyond each associated first reflector (112a–e) and being cooled by the aspirated cooling air stream, flowing outwardly past the first reflector.

11. Suntan apparatus as claimed in claim 9 or 10, characterised by an air suction passageway (119) branching off in the first housing area (107), air suction apertures (125–129) being provided in the passageway (119), the passageway being connected with the suction side of the blower (110) and into which there project the regions of the first reflectors (112) directly encompassing the Hg high pressure radiator (115).

12. Suntan apparatus as claimed in claim 11, characterised by at least one air suction aperture (125–129) being associated with each radiator (115a–e).

13. Suntan apparatus as claimed in claim 11 or 12, characterised by an ignition apparatus (130) associated with each radiator (115) in the flow of the aspirated cooling air.

14. Suntan apparatus as claimed in claim 1, characterised by the UV-filter (9) being combined of scale-like overlapping strips of blue-violet filter glass.

15. Suntan apparatus as claimed in claim 14, characterised by the filter glass strips extending in the direction of the cooling air stream flowing therepast.

**Revendications**

1. Appareil de brunissage destiné à la pigmentation directe de la peau humaine, comprenant, disposés dans un carter commun, au moins une lampe à mercure à haute pression, au moins un filtre thermique, au moins un filtre UV, un réflecteur, ainsi qu'un ventilateur pour l'alimentation en air frais, caractérisé par le fait que le carter (1, 101) est divisé en deux sections par une paroi intermédiaire (6, 106), la première section (7, 107) du carter étant reliée au côté aspiration d'air du ventilateur (12) et la deuxième section (8, 108) du carter étant reliée au côté refoulement du ventilateur (12, 110), et par le fait que la (ou les) lampe(s) à mercure sous haute pression (2, 115) sont disposées dans l'une des sections du carter, tandis que le filtre thermique (5, 132) et le filtre UV (9, 114) sont situés dans le courant d'air direct formé par l'air frais aspiré et/ou refoulé et que au moins la section de décharge (23, 118) de la lampe à mercure sous haute pression (2, 115) est protégée contre le courant de l'air frais aspiré et/ou refoulé.

2. Appareil de brunissage selon la revendication 1, caractérisé par le fait que dans le cas d'une seule lampe à mercure haute pression (2) disposée dans le carter (1), celle-ci est placée dans la deuxième section (8) dudit carter reliée au côté refoulement du ventilateur (12), par le fait que le filtre thermique (5) est disposé dans une paroi extérieure du carter (1) en recouvrant l'orifice de rayonnement de l'appareil et par le fait que cette cette paroi extérieure, qui comporte le filtre thermique, délimite sur un côté la première section (7) du carter, par le fait que le filtre UV (9) est disposé dans la paroi intermédiaire (6) et par le fait que le réflecteur (18) est subdivisé et s'étend aussi bien dans la première section (7) que dans la deuxième section (8) du carter et présente, dans sa partie (20) située dans la deuxième section (8) du carter, un orifice d'admission (21) et un orifice d'échappement (22) qui permettent le passage du courant d'air frais refoulé sur le filtre UV (9).

3. Appareil de brunissage selon la revendication 2, caractérisé par le fait que le réflecteur (18) présente, au moins dans un plan, une forme parabolique et que son orifice d'admission (21) est situé devant l'orifice de refoulement (13) du ventilateur de telle manière qu'une partie d'un courant d'air frais refoulé passe derrière le réflecteur et que la section de décharge (23) de la lampe à mercure haute pression (2) se situe dans la zone protégée du courant d'air frais entrant par l'orifice d'admission (21).

4. Appareil de brunissage selon la revendication 3, caractérisé par le fait que les pattes de pincement (3), portant les embouts de connexion électrique (4) de la lampe à mercure haute pression (2) s'étendent latéralement vers l'extérieur par-dessus le réflecteur (20) et sont refroidies par la partie du courant d'air frais qui passe derrière le réflecteur.

5. Appareil de brunissage selon les revendications 2 ou 3, caractérisé par le fait que la partie (19) du réflecteur (18), située dans la première section (7) du carter, est disposée sur la paroi latérale (6) et se termine à une certaine distance de la paroi extérieure portant le filtre thermique (5) et dégage un passage pour le courant d'air frais aspiré.

6. Appareil de brunissage selon l'une des revendications précédentes, caractérisé par le fait que le ventilateur (12) est disposé à l'intérieur du carter (1) dans une chambre (11) séparée de la deuxième section (8) du carter par une paroi de séparation (10), tandis que l'orifice (13) du ventilateur, côté refoulement, est situé dans la paroi de séparation 10 et par le fait que dans la paroi intermédiaire (6) est prévue une ouverture d'aspiration (14) entre la première section (7) du carter et la chambre (11).

7. Appareil de brunissage selon la revendication 6, caractérisé par le fait que dans la deuxième section (8) du carter sont disposées des tôles de direction (24, 25) destinées à diriger le courant d'air frais refoulé vers les fentes d'échappement (16) dans le carter (1).

8. Appareil de brunissage selon la revendication 7, caractérisé par le fait que, respectivement une tôle de direction (25) est fixée de manière réglable sur la paroi intermédiaire faisant face à l'orifice d'échappement (22) du réflecteur (20) et par le fait qu'au moins une tôle de direction (24) est fixée de manière réglable sur la paroi de séparation (10) faisant face au côté arrière du réflecteur (20).

9. Appareil de brunissage selon la revendication 1, caractérisé par le fait que, en cas de présence de plusieurs lampes à mercure haute pression (115a–e) disposées dans un carter (101), lesdites lampes sont placées en une rangée juxtaposée dans la première section (107) du carter reliée au côté d'aspiration du ventilateur (110), par le fait que le filtre thermique (132) est disposé dans une paroi extérieure (103) du carter recouvrant l'orifice de sortie du rayonnement (131) de l'appareil et par le fait que cette paroi extérieure comportant le filtre thermique délimite la section (108) du carter reliée au côté refoulement du ventilateur (110), qu'un filtre UV (114a–e) est disposé pour chacune des lampes (115a–e) dans la paroi intermédiaire (106), que chaque lampe à mercure est entourée par un premier réflecteur (112a–e) fixé au moins partiellement en forme de parabole sur une paroi intermédiaire (106), ledit réflecteur (112a–e) protégeant la section de décharge (118) de la lampe à mercure à haute pression contre le courant d'air frais aspiré, et par le fait que, à l'intérieur de la deuxième section (108) du carter, entre les filtres UV (114a–e) et le filtre thermique (132), s'étend un réflecteur (133) commun pour toutes les lampes et en forme de gouttière qui est parcouru dans le sens longitudinal par le courant d'air frais refoulé.

10. Appareil de brunissage selon la revendication 9, caractérisé par le fait que les pattes de pincement (116, 117) portant les embouts de connexion électrique de chacune des lampes à mercure haute pression (115a–e) se projettent latéralement vers l'extérieur au-dessus de chaque premier réflecteur (112a–e) correspondant et sont refroidis par le courant d'air frais aspiré passant devant le premier réflecteur à l'extérieur.

11. Appareil de brunissage selon les revendications 9 et 10, caractérisé par le fait que dans la première section (107) du carter est disposé un canal d'aspiration de l'air (119), qui présente des orifices d'aspiration (125–129) et qui est relié au côté aspiration du ventilateur (110) et dans lequel pénètrent les zones des premiers réflecteurs (112) qui entourent directement la lampe à mercure à haute pression (115).

12. Appareil de brunissage selon la revendication 11, caractérisé par le fait qu'un ou plusieurs orifices d'aspiration d'air séparés (125–129) sont subordonnés à chaque lampe (115).

13. Appareil de brunissage selon les revendications 11 ou 12, caractérisé par le fait que l'instrument d'allumage (130), faisant partie de chacune des lampes (115), est disposé à l'intérieur du canal d'aspiration de l'air (119), dans le courant d'air frais aspiré.

14. Appareil de brunissage selon l'une des revendications précédentes, caractérisé par le fait que le filtre UV (9) est composé de bandes se superposant à la manière d'écailles constituées en verre filtrant les rayons ultraviolets.

15. Appareil de brunissage selon la revendication 14, caractérisé par le fait que les bandes du verre filtrant s'étendent en direction du courant d'air frais passant devant le filtre.

FIG.1

rel. spektr. Erythem - Wirksamkeit

Wellenlänge λ

0 027 187

FIG.2

0 027 187

**FIG.3**

**FIG.4**

FIG.5

FIG.6

# FIG.7